**Europäisches Patentamt**

**European Patent Office**

**Office européen des brevets**

(19)

(11) Numéro de publication: **0 068 959**
**A2**

(12) **DEMANDE DE BREVET EUROPEEN**

(21) Numéro de dépôt: **82401066.4**

(22) Date de dépôt: **11.06.82**

(51) Int. Cl.³: **G 01 N 27/16**

(30) Priorité: **15.06.81 FR 8111738**

(43) Date de publication de la demande: **05.01.83**
**Bulletin 83/1**

(84) Etats contractants désignés: **BE DE GB IT LU NL**

(71) Demandeur: **Etablissement public dit:**
**CHARBONNAGES DE FRANCE, 9, Avenue Percler,**
**F-75008 Paris (FR)**

(72) Inventeur: **Boutonnat, Maurice, Route des Carrières,**
**F-60270 Gouvieux (FR)**
Inventeur: **Rose, Gérard, 100 rue A. Briand,**
**F-60470 Villers St Paul (FR)**

(74) Mandataire: **Chevallier, Robert, Cabinet**
**BOETTCHER 23, rue La Boétie, F-75008 Paris (FR)**

(54) **Procédé et appareil d'explosimétrie à moyen nouveau de lever de doute des mesures.**

(57) La cellule de détection (D) est raccordée à une bouteille (8) de gaz combustible ayant une valve (10) associée à un poussoir (12) qui est manœuvré grâce à un bouton supplémentaire (15) qui commande aussi la mise en action d'une source supplémentaire (III) de tension et d'un galvanomètre (6) ayant une graduation supplémentaire (6B) d'oxygènométrie, le doute sur la mesure donnée par la graduation (6A) d'explosimétrie étant levé par la mesure d'oxygénométrie.

Procédé et appareil d'explosimétrie à moyen nouveau de lever de doute des mesures.

L'invention a pour objet un explosimètre, ou éventuellement un grisoumètre, auquel est incorporé un moyen nouveau d'interprétation sans doute possible de la signification des mesures faites.

Plus précisément, l'invention concerne un explosimètre du type comprenant une cellule qui contient un élément détecteur catalytique, tel qu'un filament spécial en platine ou qu'une perle de céramique recouverte d'un catalyseur; cet élément est préchauffé par un courant électrique à une température qui provoque la combinaison du ou des corps combustibles gazeux contenus dans un volume d'air donné avec l'oxygène de cet air. Cette combinaison avec l'oxygène produit un échauffement qui fait varier la résistance électrique de l'élément détecteur et cette variation déséquilibre un montage en pont associé à un appareil de mesure électrique.

On sait que l'échauffement maximum a lieu quand le gaz combustible et l'oxygène sont présents en quantité stoechiométrique soit 9% env. dans l'air dans le cas du méthane, ce qui correspond à un volume de méthane pour deux volumes d'oxygène. A des teneurs différentes, que ce soit à des valeurs inférieures ou à des valeurs supérieures, l'échauffement est moins important et il l'est d'autant moins que l'on s'écarte davantage du rapport stoechiométrique. Il en résulte que l'appareil de mesure électrique d'un explosimètre donne une même indication pour deux valeurs très différentes de la concentration de l'atmosphère en gaz combustible. L'une de ces valeurs est inférieure à la teneur stoechiométrique, l'autre lui est supérieure. La lecture de l'indication donnée par l'appareil comporte donc une ambiguïté et il est nécessaire de lever le doute afin de pouvoir interpréter correctement la mesure faite.

On connaît plusieurs moyens dont le rôle est de lever le doute, qui sont employés sur les explosimètres et principalement sur les grisoumètres.

Dans le brevet français n° 1 442 430, au nom du déposant, qui concerne un grisoumètre, l'appareil de mesure électrique est un galvanomètre dont le cadre mobile est associé à deux aiguilles décalées angulairement d'un angle pouvant être de 120° et qui se déplacent devant un même cadran gradué.

Dans le brevet français n° 1 446 363, également au nom du déposant, le pont de mesure est alimenté périodiquement en courant continu par commutation commandée par une base de temps réglable; le déséquilibre du pont donne naissance à chaque coupure de l'alimentation à un signal de déséquilibre dont la variation est amplifiée et sert à déclencher un organe d'alarme ou de protection.

Ces deux moyens reposent sur l'observation faite que certains ponts de mesure fournissent systématiquement une tension de déséquilibre dont la polarité s'inverse quand la teneur en grisou dépasse 8%. On pourra se reporter aux brevets cités ci-dessus pour prendre connaissance de la constitution des grisoumètres en question et notamment de leur complexité.

L'invention a pour but principal d'apporter un procédé et un explosimètre nouveaux en ce sens qu'ils comportent un moyen nouveau, de réalisation moins coûteuse et valable pour tous les gaz combustibles, pour lever le doute des mesures faites, reposant sur l'exploitation d'un phénomène autre que l'inversion du signal de déséquilibre.

Un but secondaire de l'invention est de parvenir à un explosimètre défini ci-dessus dans lequel le moyen nouveau servant à lever le doute est susceptible de fournir, en plus, une information utile relative à la teneur en oxygène de l'air chargé de gaz combustible, que les explosimètres équipés des moyens connus pour lever le doute sont incapables de donner.

Selon le procédé de l'invention, on mesure la teneur d'une atmosphère en gaz combustible au moyen d'un explosimètre connu en soi comprenant une cellule et un appareil de mesure électrique qui fournit une indication ambiguë.S1 dont le doute n'est pas levé, on conserve en mémoire cette indication S1 fournie par l'appareil de mesure électrique, on procède aussitôt à une seconde mesure de la même atmosphère en injectant dans la cellule un gaz combustible à un débit qui conduit à un dépassement de la concentration stoechiométrique, on observe l'indication maximale S2 fournie par l'appareil de mesure électrique et on compare les indications S1 et S2. Quand S2 a une valeur supérieure à S1, on en déduit que la concentration en gaz combustible était inférieure à la teneur stoechiométrique. Quand S2 n'est pas la valeur maximale d'une indication croissante dépassant S1 mais une indication égale à S1 et décroissante aussitôt, on en déduit que la concentration en gaz combustible était supérieure à la teneur stoechiométrique. De cette façon le doute est levé d'une manière claire sur l'interprétation à faire de la première mesure S1.

Le procédé de l'invention que l'on vient de définir se met en oeuvre à l'aide d'un explosimètre classique auquel on a ajouté un réservoir de gaz combustible auxiliaire et les éléments appropriés de commande et de contrôle nécessaires à l'exécution des deux mesures successives et à la comparaison de leurs résultats S1 et S2.

En fait, la seconde mesure qui fournit l'indication S2 est représentative de la teneur en oxygène de l'atmosphère soumise à la mesure quand cette valeur S2 est une valeur maximale d'un signal qui est croissant à partir d'une valeur inférieure égale à S1. On trouvera des explications détaillées sur la mesure de la teneur en oxygène d'un mélange gazeux en se reportant au brevet français n° 76 30245 (2 367 285 ) du déposant. Ce brevet décrit un procédé et un appareil qui sont consacrés exclusivement à la mesure de la teneur en oxygène.

La présente invention consiste à avoir combiné ensemble le procédé et l'appareil classiques de mesure de la teneur en gaz combustible et le procédé et l'appareil du brevet cité ci-dessus de mesure de la teneur en oxygène afin de parvenir à un procédé et un appareil uniques qui, dans un premier temps, procurent une indication univoque de la teneur en gaz combustible, comme on l'a expliqué plus haut, et qui, dans un deuxième temps, procurent en plus une indication sur la teneur en oxygène ainsi qu'on l'expliquera maintenant.

En effet, si en plus de comparer dans un circuit électronique les indications S1 et S2 pour connaître seulement le résultat de la comparaison qui lève le doute sur l'interprétation de S1, on fait apparaître lisiblement la valeur de S2 en tant que valeur maximale d'un signal croissant, on obtient par cette valeur S2 une indication sur la teneur en oxygène de l'atmosphère dont on vient de mesurer sans ambiguïté la teneur en gaz explosif.

En pratique, un appareil mettant en oeuvre le procédé de l'invention peut être réalisé selon plusieurs modes différents selon que l'on désire obtenir soit un explosimètre à lever de doute dont l'utilisation peut être manuelle ou peut être rendue automatique, soit un explosimètre à lever de doute - oxygénomètre dont l'utilisation peut elle aussi être manuelle ou peut être rendue automatique.

Dans l'un et l'autre cas, la transformation de l'utilisation manuelle en fonctionnement automatique se fait à l'aide de circuits électroniques appropriés qui sont combinés en vue de procurer le résultat recherché. Il ne semble pas nécessaire de décrire ici les quatre versions possibles énumérées ci-dessus de l'appareil de l'invention. On se limitera dans ce qui suit à la description de l'appareil le plus simple : l'explosimètre à lever de doute et à utilisation manuelle - qui se distingue des explosimètres connus par ses organes de lever de doute - et à la description de l'appareil le plus complexe : l'explosimètre à lever de doute-oxygénomètre à fonctionnement automatique. Ce dernier comprend des moyens matériels nouveaux inexistants sur les appareils connus jusqu'à présent d'explosimétrie et d'oxygénométrie; ces moyens sont principalement :

- un appareil de mesure électrique à deux échelles graduées dont l'une se rapporte à la teneur en gaz explosif et l'autre à la teneur en oxygène,

- deux boutons distincts, l'un pour la commande de la première mesure de la teneur en gaz explosif, l'autre pour la seconde mesure de la teneur en oxygène,

- un circuit électronique qui est capable au moins de la mémorisation de la valeur S1 et de la comparaison de celle-ci à la valeur S2.

Toutefois, on expliquera, en plus, comment on peut réaliser d'autres appareils intermédiaires entre les deux appareils décrits en détail.

De préférence, dans les modes de réalisation de l'invention, l'appareil comprend aussi des moyens de modification de la tension électrique d'alimentation du pont en fonction de l'exécution de la première ou de la seconde mesure, une tension plus élevée (1,3 V par exemple) étant souhaitable pour la première mesure et une tension moins élevée (1,1 V par exemple) étant suffisante pour la seconde mesure quand on désire connaître la teneur en $O_2$ de l'atmosphère.

Au cours de la description qui suit on se reportera aux dessins annexés dans lesquels :

- la figure 1 est un schéma électrique d'un explosimètre à lever de doute selon l'invention, à utilisation manuelle,

- la figure 2 est un schéma électrique d'un explosimètre à lever de doute-oxygénomètre selon l'invention, à fonctionnement automatique,

- les figures 3 et 4 sont respectivement des schémas d'appareils intermédiaires entre ceux des figures 1 et 2.

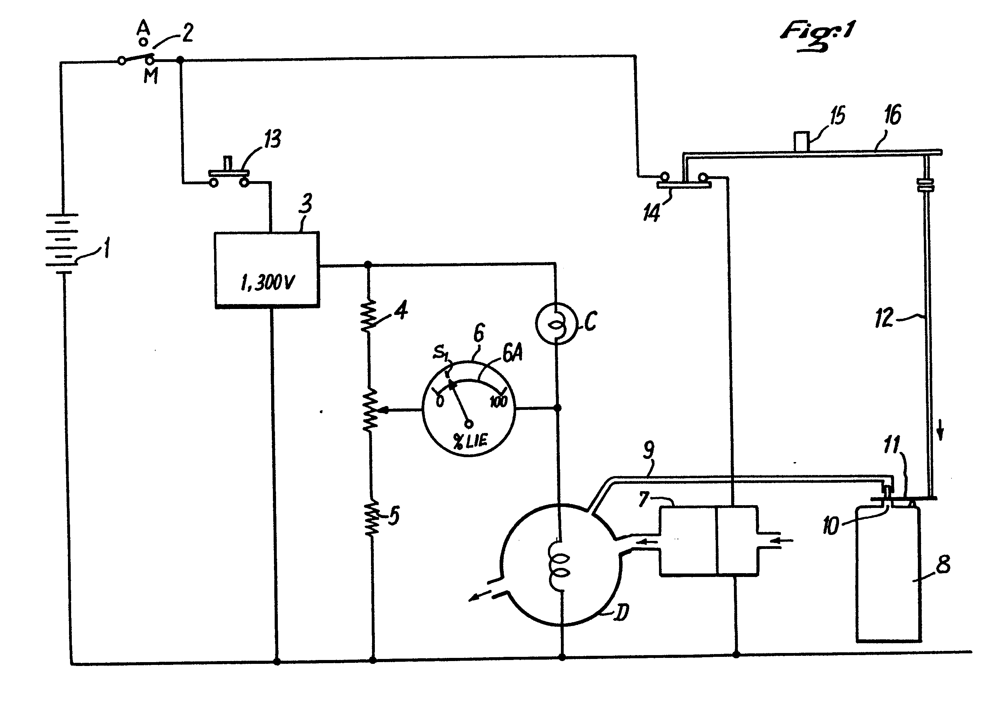

Un explosimètre à lever de doute simple dont le schéma est donné par la figure 1 comprend une source 1 de courant électrique associée à un interrupteur général Marche-Arrêt 2 pour alimenter un bloc de régulation 3 qui fournit une tension stabilisée à 1,3 V. Ce bloc 3 est destiné à alimenter un montage en pont qui comprend des résistances 4, 5 ainsi qu'une première cellule de détection D à filament détecteur et une seconde cellule C à filament compensateur. Ce montage connu comprend encore un galvanomètre 6, à réglage de la valeur zéro, qui a une graduation 6A de O à 100% représentant le pourcentage de la limite inférieure d'explosivité que l'on appellera plus loin L.I.E.. La cellule de détection D a deux ouvertures opposées dont l'une est raccordée à une pompe 7 à moteur électrique alimenté par la source 1. Cette pompe 7 aspire l'atmosphère environnant et réalise le balayage de la cellule D. L'explosimètre comprend encore, spécifiquement pour le lever de doute, une bouteille 8 de gaz combustible sous pression - de préférence du butane- dont l'orifice de sortie est raccordé par une tubulure 9 au volume intérieur de la cellule D. La bouteille 8 est munie d'une valve de fermeture 10 qui s'ouvre sous l'action d'un levier 11 associé à un poussoir 12.

Entre l'interrupteur général 2 et le bloc de régulation 3 est placé un bouton-poussoir de mesure 13 qui est normalement ouvert; entre le même interrupteur général 2 et le moteur de la pompe 7 est placé un autre bouton-poussoir à contacts 14 qui sont normalement fermés. Ce bouton-poussoir fait partie d'un bouton 15 de lever de doute qui est apte à agir aussi sur le poussoir 12. En pratique le bouton 15 de lever de doute est pourvu de préférence d'une barrette 16 dont une extrémité agit sur le bouton-poussoir 14 pour ouvrir celui-ci et dont l'extrémité opposée agit sur le poussoir 12 dans le sens de l'ouverture de la valve 10, quand on enfonce ce poussoir 15 contre l'effet d'un moyen de rappel (non représenté).

Sur un explosimètre de ce type, on trouve rassemblés sur une même face, de préférence la face supérieure, l'interrupteur général 2, le bouton-poussoir de mesure 13, le bouton de lever de doute 15, le galvanomètre 6. L'utilisation se fait manuellement de la façon suivante.

Dès la fermeture de l'interrupteur général 2, la pompe 7 envoie à travers la cellule de détection D un courant de l'atmosphère environnante; on appuie sur le bouton-poussoir de mesure 13, on lit alors sur le cadran du galvanomètre une valeur S1 indiquée par l'aiguille. En continuant à appuyer sur le bouton-poussoir de mesure 13 et en observant l'aiguille du galvanomètre, on enfonce le bouton de lever de doute 15. Simultanément, les contacts 14 s'ouvrent, la pompe 7 s'arrête, la valve 10 s'ouvre et du butane arrive par la tubulure 9 dans la cellule de détection D. Deux cas sont possibles à ce moment :     a) l'aiguille monte à partir de S1 et atteint une valeur supérieure S'. On en déduit que la valeur S1 était correcte et indiquait la valeur en % L.I.E. de la teneur de l'atmosphère en gaz explosif ,

b) l'aiguille ne monte pas mais descend à partir de S1. On en déduit que la valeur S1 ne représentait pas le % L.I.E. et que, au contraire, la teneur de l'atmosphère en gaz explosif est supérieure à L.I.E..

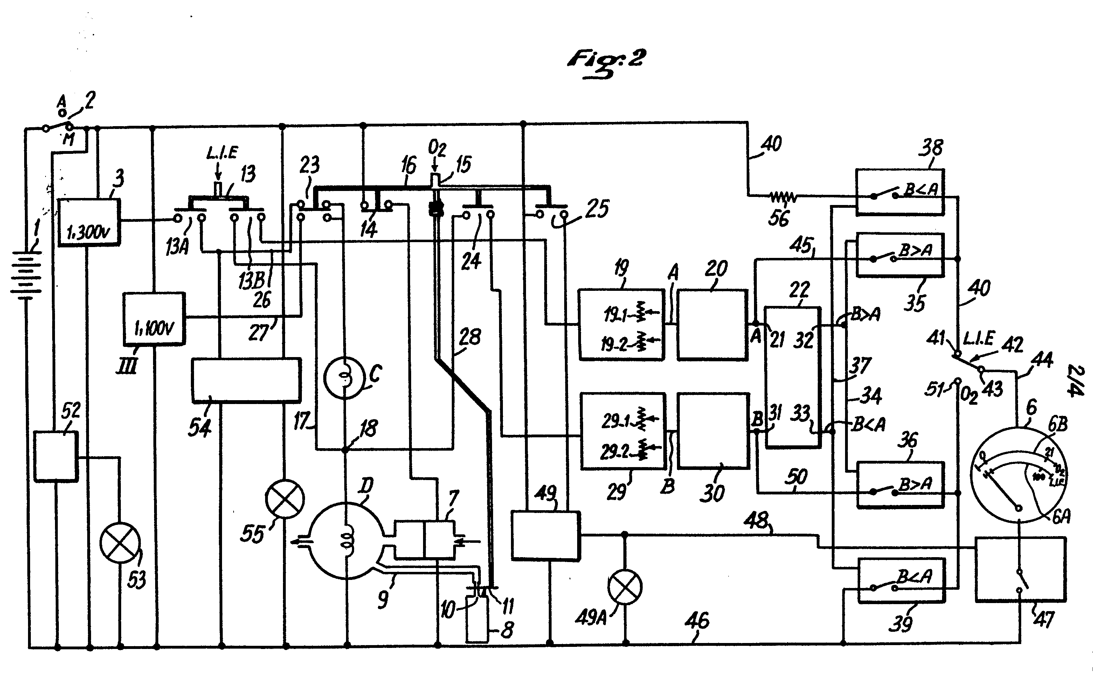

L'appareil dont le schéma est donné par la figure 2 est plus complexe d'une part parce qu'il indique séparément, en plus de la valeur S1, la valeur S2 qui représente la teneur de l'atmosphère en % $O_2$, d'autre part parce qu'il est à fonctionnement automatique. Mais les organes de base selon l'invention sont les mêmes de sorte que l'on utilisera les mêmes références pour désigner les pièces communes avec l'appareil de la figure 1 et que l'on ne répétera pas la description de la partie commune avec cet appareil. Les différences par rapport à celui-ci sont les suivantes.

En plus du bloc de régulation 3 fournissant une tension de 1,3 V pour l'explosimétrie, il existe un autre bloc de régulation III fournissant une tension de 1,1 V pour l'oxygénométrie. Le bouton-poussoir 13 est pourvu de deux paires de contacts normalement ouverts, une paire 13A pour l'alimentation en 1,3 V à sa fermeture de l'ensemble du pont, une paire 13B qui est placée sur une ligne 17 qui relie un point 18 entre les cellules C et D à l'entrée d'un amplificateur 19 du signal de mesure d'explosimétrie. Cet amplificateur 19 est de préférence à gain réglable 19-1 et à réglage de zéro 19-2; il est suivi d'un circuit 20 de mise en mémoire du signal indiqué par la lettre A qu'il transmet aussi à une entrée 21 d'un comparateur 22.

La barrette 16 du bouton 15 est allongée pour agir sur les contacts 14 normalement fermés associés à la pompe 7, sur le poussoir 12 de la bouteille 8 de gaz combustible et, en plus, sur un commutateur-inverseur 23 et les éléments mobiles de fermeture de contacts 24 et de contacts 25. Le commutateur-inverseur 23 a une première position normale dans laquelle il ferme la ligne 26 d'alimentation en 1,3 V des cellules C, D et une seconde position à l'enfoncement du bouton 15 dans laquelle il ouvre la ligne à 1,3 V et ferme une seconde ligne 27 provenant du bloc de régulation III pour alimenter en 1,1 V les cellules C, D. Les contacts 24, normalement ouverts, sont situés sur une ligne 28 qui relie le point 18 entre les cellules C et D à l'entrée d'un amplificateur 29 du signal de mesure de la teneur en oxygène de de l'atmosphère environnante. Cet amplificateur 29 est de préférence à gain réglable 29-1 et à réglage de zéro 29-2; il émet un signal B qu'il fournit à un circuit 30 de mise en mémoire de ce signal qui est transmis en outre à une autre entrée 31 du comparateur 22. Ce dernier a deux sorties 32, 33 qui sont reliées respectivement par une ligne 34 à une entrée de commande de deux interrupteurs analogiques 35, 36, et par une ligne 37 à une entrée de commande de deux autres interrupteurs analogiques 38, 39.

L'interrupteur 38 est placé sur une ligne générale 40 provenant de l'interrupteur général 2 et aboutissant à une borne 41 de signal L.I.E d'un commutateur 42 à deux positions ayant une borne centrale 42 reliée par un conducteur 44 à une borne du galvanomètre 6.

L'interrupteur 35 est placé sur une ligne 45 qui relie l'entrée 21 du comparateur 22 à la borne 41 du commutateur 42.

L'interrupteur 39 est placé sur une ligne générale 46 qui part d'une borne de la source 1 pour aboutir à la seconde borne du galvanomètre 6 à travers un interrupteur analogique 47 dont l'entrée de commande est réunie par une ligne 48 à une sortie d'un circuit de temporisation 49 qui est lui-même branché entre les lignes générales 40, 46 et raccordé aux contacts 25 normalement ouverts associés à la barrette 16 du bouton 15. Une ampoule de signalisation 49A est branchée entre les lignes 48 et 46.

L'interrupteur 36 est placé sur une ligne 50 qui relie l'entrée 31 du comparateur 22 à une borne 51 de signal $O_2$ du commutateur 42.

L'appareil comprend encore un circuit 52 de temporisation de balayage de la cellule de détection D par l'atmosphère soufflée par la pompe 7. Ce circuit 52 est branché entre les lignes générales 40, 46 et associé à une ampoule de signalisation 53. Un autre circuit 54 de temporisation de la mesure L.I.E. est branché entre la ligne 26 et la ligne générale 46; il est associé à une lampe de signalisation 55.

Les blocs de régulation 3 et III sont montés entre les lignes générales 40, 46 ainsi que le bouton poussoir à contacts 14.

Le circuit qui vient d'être décrit comprend deux parties qui partent toutes deux de la source 1 et aboutissent au galvanomètre 6. Une première partie met en service le bloc de régulation 3 à 1,3 V grâce au bouton poussoir 13 pour fournir à travers l'amplificateur 19 et à travers le circuit 20 de mise en mémoire un signal A au comparateur 22; ce signal A représente la mesure de la teneur de l'atmosphère en gaz explosif. La seconde partie du circuit met en service le bloc de régulation III à 1,1 V grâce au bouton 15 pour fournir à travers l'amplificateur 29 et à travers le circuit 30 un signal dont la valeur de crête B est mise en mémoire et transmise au comparateur 22; cette valeur B représente la mesure de la teneur de l'atmosphère en oxygène.

Le fonctionnement de l'appareil est le suivant.

Dès la fermeture de l'interrupteur général 2, la pompe 7 envoie un courant de l'atmosphère environnante qui balaie la cellule de détection D. Quand la lampe 53 s'allume, le balayage a été suffisamment long, on peut appuyer sur le bouton 13 de mesure L.I.E.. Il en résulte le signal A dont la valeur est conservée en mémoire dans le circuit 20 et fournie au comparateur 22. Quand l'ampoule 55 s'allume, on peut procéder à l'opération suivante en lâchant le bouton 13 et en appuyant sur le bouton 15 de mesure de $O_2$. Il se produit, simultanément, le changement de tension de 1,3 à 1,1 V, l'arrêt de la pompe 7, l'ouverture de la valve 10 de la bouteille 8, l'envoi du signal de mesure à l'amplificateur 2,9, la mise en action du circuit de temporisation 49 de la mesure de $O_2$. Il en résulte le signal dont la valeur maximale ou valeur de crête B est conservée en mémoire dans le circuit 30 et fournie au comparateur 22.

Jusqu'à cet instant, le galvanomètre 6 est resté inactif.

Quand le circuit de temporisation 49 de mesure de $O_2$ le permet, l'ampoule 49A s'allume et l'interrupteur analogique 47 se ferme autorisant la mise en service du galvanomètre 6. A ce moment, à la sortie du comparateur 22, si on a B > A, les interrupteurs 35 et 36 sont fermés et les interrupteurs 38 et 39 restent ouverts. Quand le commutateur 42 est mis à la position L.I.E. de fermeture sur la borne 41, l'aiguille du galvanomètre 6 indique sur la graduation 6A la valeur $S_1$ qui est le pourcentage exact de L.I.E. du gaz explosif dans l'atmosphère, valeur transmise à travers l'interrupteur 35. Quand le commutateur 42 est mis à la position $O_2$ de fermeture sur la borne 51, l'aiguille indique sur une graduation 6B la valeur $S_2$ qui est le pourcentage de $O_2$ dans l'atmosphère, valeur transmise à travers l'interrupteur 36.

Au contraire, si à la sortie du comparateur 22 on a B < A, les interrupteurs 35 et 36 restent ouverts et les interrupteurs 38 et 39 sont fermés. Quand le commutateur 42 est mis à la position L.I.E. sur la graduation 6A, l'aiguille se déplace au-delà de la valeur 100% pour indiquer que la teneur en gaz explosif dépasse la L.I.E., puisque le galvanomètre 6 est directement réuni à la source 1, à part une résistance de protection 56, par les lignes générales 40, 56. Quand le commutateur 42 est mis à la position $O_2$, sur la graduation 6B l'aiguille reste en dessous de la valeur 0, pour indiquer que la mesure du pourcentage de $O_2$ est inexacte, puisque le galvanomètre 6 est mis en court-circuit par l'interrupteur 39.

On peut réaliser des appareils intermédiaires à ceux qui ont été décrits ci-dessus, à partir du circuit de base de l'appareil de la figure 1, en y ajoutant une partie des circuits de l'appareil de la figure 2.

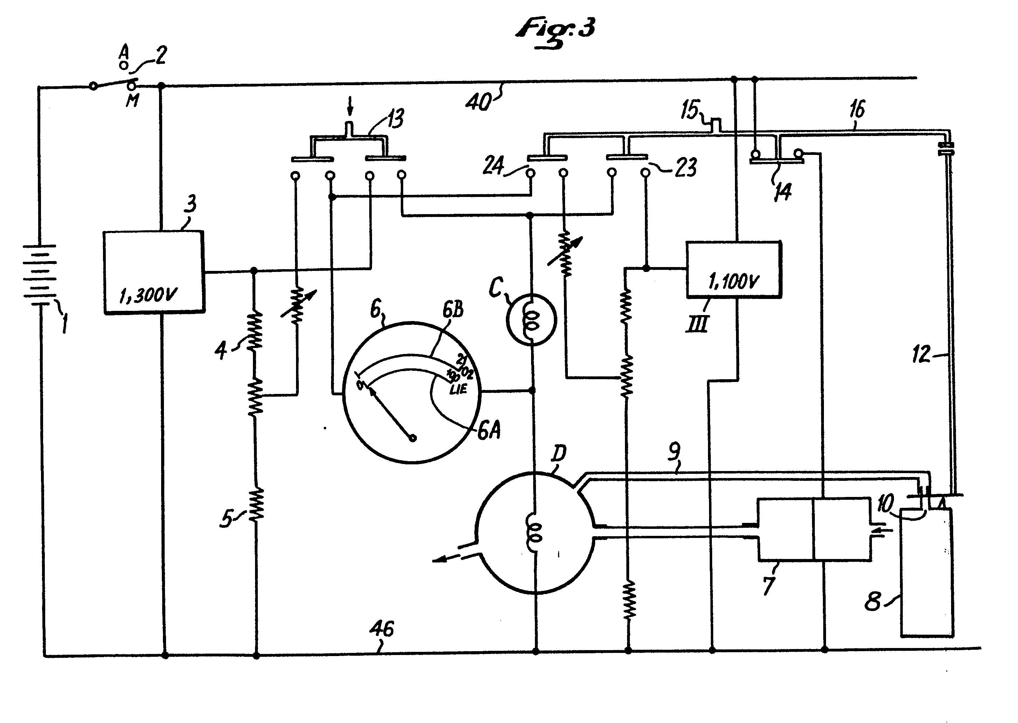

Par exemple, on peut parvenir à un explosimètre avec lever de doute simple-oxygénomètre à utilisation manuelle en retirant du schéma de la figure 2 les amplificateurs 19, 29, les circuits de mise en mémoire 20, 30, le comparateur 22, les interrupteurs 35, 36, 38, 39, 47. On peut supprimer aussi les circuits de temporisation 52, 54, 49 si on veut laisser à l'utilisateur l'appréciation du temps à laisser s'écouler entre les diverses opérations de mesure. Un tel appareil dont le schéma est donné sur la figure 3 comprend le bouton-poussoir 13 de la figure 2 avec les deux blocs de régulation 3 et III, le bouton 15 avec la barrette 16 agissant sur le commutateur-inverseur 23 pour le changement de tension, sur le bouton poussoir à contacts 14 pour la marche de la pompe 7, sur le poussoir 12 de la valve 10, et sur des contacts 24 pour brancher directement le galvanomètre 6 fonctionnant sur la seconde graduation 6B pour la lecture du pourcentage de $O_2$.

La différence avec le circuit de la figure 1 est que le galvanomètre 6 a la double graduation 6A, 6B, qu'il existe deux blocs de régulation 3 et III à 1,3 V et à 1,1 V, commutables, et un branchement sur le pont du galvanomètre 6 par le moyen du bouton 15 pour la lecture de la valeur $S_2$, valeur qui n'est pas connue avec l'appareil de la figure 1 puisque la graduation 6B n'existe pas sur le galvanomètre de cet appareil. Comme avec ce dernier, la valeur $S_1$ n'est pas mise en mémoire dans un circuit électronique. C'est l'utilisateur qui retient la valeur de $S_1$ sur une graduation 6A, qui observe le déplacement de l'aiguille sur cette graduation et qui lit la valeur de $S_2$ sur la seconde graduation 6B.

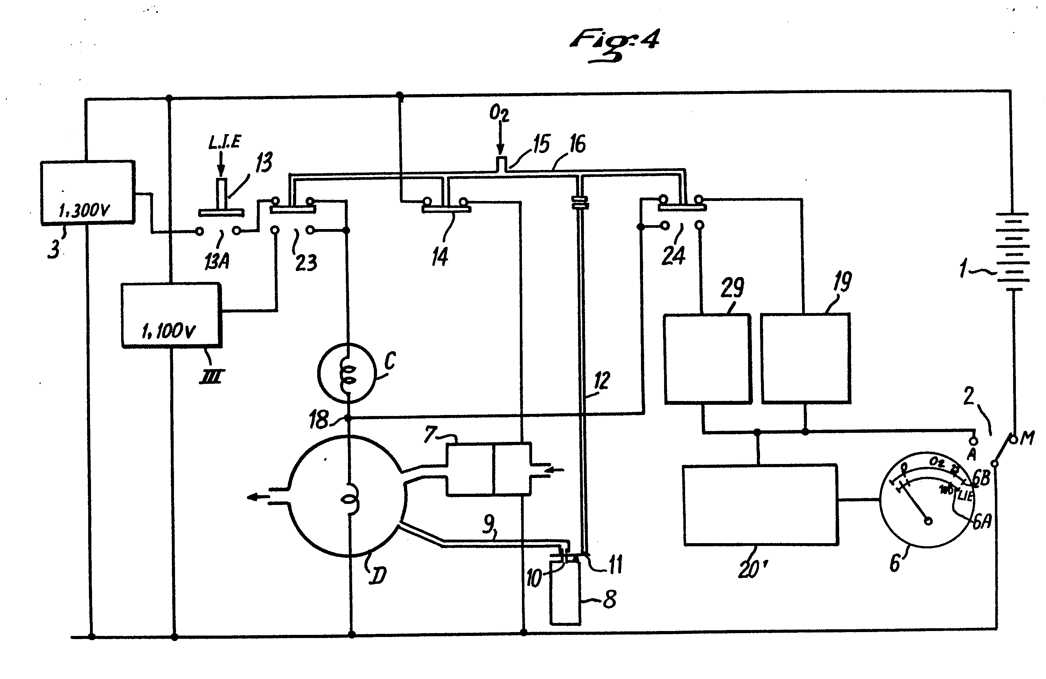

A partir de l'appareil intermédiaire que l'on vient de décrire, on peut obtenir un explosimètre à lever de doute simple-oxygénomètre avec mise en mémoire de l'une des mesures, dont le schéma est donné sur la figure 4. Dans ce cas, il suffit de reprendre les amplificateurs 19, 29 de la figure 2 raccordés ensemble à un unique circuit 20' de mise en mémoire du signal maximum qu'il reçoit. A ce circuit 20' est relié le galvanomètre 6 à deux graduations 6A, 6B. L'utilisateur n'a pas besoin comme avec l'appareil de la figure 1 de maintenir appuyé le bouton 13 quand il enfonce le bouton 15. La manoeuvre du bouton 13 fait apparaître à la sortie de l'amplificateur 19 le signal A qui est retenu dans le circuit à mémoire 20' et qui se traduit par l'indication de la valeur $S_1$ sur la graduation 6A du galvanomètre 6. Le circuit à mémoire 20' maintient l'aiguille à cette position quand on appuie sur le bouton 15 qui fait apparaître à la sortie de l'amplificateur 29 le signal B. Si on a B > A, l'aiguille monte et se stabilise pour indiquer la valeur $S_2$ sur la graduation 6B. Si on a B < A, l'aiguille ne bouge pas; on ne connaît donc pas la valeur de $S_2$ et on sait que $S_1$ n'était pas la valeur exacte de la teneur du gaz explosif en % de L.I.E..

Il est entendu que l'invention couvre toutes les variantes que l'on peut apporter aux appareils décrits ci--dessus et qui appliqueraient le procédé de lever de doute de l'invention. Par exemple, on parvient facilement à un explosimètre à lever de doute automatique à partir du schéma de la figure 2. Il suffit de supprimer les composants qui se rapportent à la lecture de la teneur de l'atmosphère en oxygène, c'est-à-dire l'amplificateur 29, le circuit de mise en mémoire 30, de supprimer aussi les interrupteurs 36 et 39, et d'utiliser un galvanomètre 6 à simple graduation 6A en % L.I.E.. Dans ce cas le signal A parvient au comparateur 22 à travers le circuit de mise en mémoire 20 associé si nécessaire à l'amplificateur 19. Le signal B parvient au comparateur 22 directement du point 18 à travers les contacts 24, le circuit 30 de mise en mémoire de la valeur de crête du signal B pouvant être conservé en amont du comparateur 22 si on le juge nécessaire. Pendant le fonctionnement, l'interrupteur 35 est fermé par le comparateur 22 quand on a $B > A$ et le galvanomètre 6 indique la valeur $S_1$ sur son unique graduation 6A; l'interrupteur 38 est fermé par le comparateur 22 quand on a $B < A$ et le galvanomètre 6 indique une valeur supérieure à 100% sur son unique graduation 6A.

Dans la description qui précède, comme le gaz contenu dans l'atmosphère et le gaz que l'on envoie dans la cellule de détection D sont tous deux des gaz combustibles, on a appelé explosif le premier gaz et combustible le second gaz pour la clarté des explications mais il doit être entendu que ceci n'implique rien sur la différence de nature de ces gaz.

## REVENDICATIONS

1. Procédé pour lever le doute de la mesure avec un explosimètre à cellule de détection de la teneur d'une atmosphère en gaz explosif donnant une indication $S_1$ caractérisé en ce qu'on procède à une seconde mesure en injectant dans la cellule de détection un gaz combustible afin d'obtenir une seconde indication $S_2$, on compare les indications $S_1$ et $S_2$, quand $S_2$ est supérieure à $S_1$ on en déduit que cette dernière exprimait la teneur en gaz explosif à une valeur inférieure au rapport stoechiométrique de ce gaz dans l'atmosphère.

2. Procédé selon la revendication 1 caractérisé en ce qu'on utilise comme gaz combustible du butane.

3. Procédé selon la revendication 1 caractérisé en ce qu'on retient la valeur $S_2$ comme exprimant la teneur de l'atmosphère en oxygène quand elle est supérieure à $S_1$.

4. Explosimètre pour la mise en oeuvre du procédé selon la revendication 1, comprenant une source (3) de tension appropriée, une cellule de détection (D) associée à une pompe de balayage (7), un galvanomètre (6) ayant une graduation (6A) appropriée, un bouton de mesure (13), caractérisé en ce qu'il comprend aussi une bouteille de gaz combustible sous pression (8) ayant une valve (10) reliée par une tubulure (9) à la cellule (D) et associée à un poussoir de manoeuvre (12) apte à être mû par un bouton supplémentaire (15) accessible à l'extérieur de l'explosimètre pour l'exécution de la seconde mesure.

5. Explosimètre selon la revendication 4 caractérisé en ce que le bouton (15) est apte à agir à la fois sur le poussoir (12) de la valve (10) et sur un bouton-poussoir à contacts (14) d'alimentation de la pompe (7) afin d'arrêter celle-ci pendant l'exécution de la seconde mesure.

6. Explosimètre selon la revendication 5 caractérisé en ce qu'il comprend au moins un circuit (20) de mise en mémoire d'un signal (A) obtenu lors de la première mesure, d'un comparateur (22) recevant et comparant le signal (A) venant du circuit (20) à un signal (B) obtenu lors de la seconde mesure, deux interrupteurs analogiques (35, 38) réunis à une borne du galvanomètre (6) et raccordés respectivement à une sortie (32, 33) du comparateur (22) pour être commandés par celui-ci en fonction de la comparaison, l'interrupteur (35) étant relié à l'entrée (21) du signal A dans le comparateur (22) et l'interrupteur (38) étant relié à la source de tension.

7. Explosimètre selon la revendication 6 caractérisé en ce qu'un circuit supplémentaire (30) de mise en mémoire de la valeur de crête du signal (B) est disposé pour recevoir ce signal et le transmettre à une entrée (31) du comparateur (22).

8. Explosimètre selon la revendication 5 caractérisé en ce qu'il comprend un amplificateur (19) recevant un signal (A) obtenu lors de la première mesure, un amplificateur (29) recevant un signal (B) obtenu lors de la seconde mesure, un circuit (20') de mise en mémoire du signal de valeur maximum (A ou B), le galvanomètre (6) étant relié à ce circuit (20').

9. Explosimètre-oxygénomètre pour la mise en oeuvre du procédé selon la revendication 3, comprenant une source (3) de tension appropriée pour la mesure d'explosimétrie, une cellule de détection (D) associée à une pompe de balayage (7), un galvanomètre (6) ayant une graduation (6A) appropriée à la mesure d'explosimétrie, un bouton de mesure (13), caractérisé en ce qu'il comprend aussi une bouteille de gaz combustible sous pression (8) ayant une valve (10) reliée par une tubulure (9) à la cellule (D) et associée à un poussoir de manoeuvre (12), un bouton supplémentaire (15), une source (III) de tension appropriée à la mesure d'oxygénométrie, le galvanomètre (6) ayant une graduation supplémentaire (6B) appropriée à la mesure d'oxygénométrie, le bouton (15) étant apte à agir simultanément sur un commutateur-inverseur (23) de changement de source de tension, sur le poussoir (12), sur des contacts (24) d'un circuit comprenant le galvanomètre (6) branché pour la lecture sur la graduation supplémentaire (6B) lors de la seconde mesure.

10. Appareil selon la revendication 9 caractérisé en ce que le circuit comprenant les contacts (24) est constitué par un premier amplificateur (19) donnant un signal (A) lors de la première mesure, relié à un circuit (20) de mise en mémoire de ce signal (A), cet amplificateur (19) étant mis en service grâce à des contacts (13A) du bouton (13), un second amplificateur (29) mis en service par les contacts (24) et donnant un signal (B) lors de la seconde mesure, un circuit (30) de mise en mémoire de ce signal (B), un comparateur (22) réuni aux deux amplificateurs (19, 29), deux interrupteurs (35, 36) reliés chacun à une sortie (32, 33) respectivement du comparateur (22) pour être fermés quand $B > A$, l'interrupteur (35) étant monté entre l'entrée du comparateur (22) et le galvanomètre (6) pour la lecture sur la graduation (6A) d'explosimétrie, l'interrupteur (36) étant monté entre l'entrée du comparateur (22) et le galvanomètre (6) pour la lecture sur la graduation (6B) d'oxygénométrie.

11. Appareil selon la revendication 10 caractérisé en ce que deux interrupteurs supplémentaires (38, 39) sont reliés chacun à une sortie (33, 32) respectivement du comparateur (22) pour être fermés quand $B < A$, l'interrupteur (38) étant monté entre la source de tension (3) et le galvanomètre (6) pour la lecture sur la graduation (6A) d'explosimétrie, l'interrupteur (39) étant monté entre les deux bornes du galvanomètre (6) pour la lecture sur la graduation (6B).

Fig.1

Fig.2

# Fig. 3

Fig.4